Europäisches Patentamt

European Patent Office

Office européen des brevets

(11) Numéro de publication: **0 346 968**
**A2**

(12) # DEMANDE DE BREVET EUROPEEN

(21) Numéro de dépôt: **89201442.4**

(51) Int. Cl.⁴: **G01N 33/12**

(22) Date de dépôt: **06.06.89**

(30) Priorité: **14.06.88 FR 8808055**

(43) Date de publication de la demande:
**20.12.89 Bulletin 89/51**

(84) Etats contractants désignés:
**DE FR GB IT NL**

(71) Demandeur: **CENTRE NATIONAL DE LA RECHERCHE SCIENTIFIQUE (CNRS)**
**15, Quai Anatole France**
**F-75700 Paris Cedex 07(FR)**

Demandeur: **CENTRE TECHNIQUE CUIR CHAUSSURE MAROQUINERIE**
**4 rue Hermann Frenkel**
**F-69367 Lyon Cédex 07(FR)**

(72) Inventeur: **Lacabanne, Colette**
**32, rue Beausoleil**
**F-31500 Toulouse(FR)**
Inventeur: **Mouthon, Gilbert**
**135, rue Gabriel Péri**
**F-94430 Chennevières(FR)**
Inventeur: **Lamure, Alain**
**34, rue du Lot Appt 275**
**F-31100 Toulouse(FR)**
Inventeur: **Dandurand, Jany**
**46, Rue Benjamin Baillaud**
**F-31500 Toulouse(FR)**

(74) Mandataire: **Barre, Philippe**
**Cabinet Barre-Gatti-Laforgue 95 rue des Amidonniers**
**F-31069 Toulouse Cédex(FR)**

(54) **Procédé de caractérisation des protéines d'un tissu musculaire.**

(57) L'invention concerne un procédé de caractérisation des protéines d'un échantillon de tissu musculaire en vue de détecter une éventuelle absorption antérieure de drogue ayant entraîné une altération desdites protéines. Le procédé consiste à disposer l'échantillon dans une cellule (1) de mesure de courant thermostimulé entre les armatures d'un condensateur (2) et à lui appliquer un champ électrique en vue de mettre en oeuvre une méthode de spectroscopie par courant thermostimulé, de façon à détecter un éventuel décalage du pic de dépolarisation irréversible qui apparaît sur la courbe de dépolarisation. La température de ce pic est abaissée d'environ 40° et son amplitude divisée par un facteur de l'ordre de 4 à 10 dans le cas d'un tissu hormoné par rapport à un tissu normal de même nature. Des courbes d'étalonnage effectuées avec des tissus normaux permettent de détecter ce décalage.

Fig. 1

Atmosphère controlée

Azote liquide

Vide

## PROCEDE DE CARACTERISATION DES PROTEINES D'UN TISSU MUSCULAIRE

L'invention concerne un procédé de caractérisation des protéines d'un tissu musculaire en vue de détecter une éventuelle altération de celles-ci. L'invention peut en particulier s'appliquer dans les services vétérinaires des abattoirs afin de contrôler la qualité des viandes abattues.

Il est essentiel de pouvoir contrôler la qualité des viandes livrées à la consommation et en particulier de détecter d'éventuelles injections hormonales chez les animaux, puisque les viandes hormonées sont actuellement soumises à des restrictions légales notamment en FRANCE. Aucun procédé efficient n'est actuellement disponible pour effectuer ce type de caractérisation. Des travaux ont montré que ni les techniques biochimiques classiques (analyse des acides aminés), ni les techniques d'études spectroscopiques localisées (telles que la résonance magnétique nucléaire RMN), ni les techniques d'études morphologiques (microscopie, technique mécanique classique...) ne fournissent des résultats significatifs permettant de différencier des viandes normales et des viandes hormonées.

La présente invention se propose d'indiquer un nouveau procédé de caractérisation des protéines d'un tissu musculaire en vue de détecter une éventuelle absorption antérieure de drogue ayant entraîné altération desdites protéines.

Un objectif de l'invention est d'indiquer un procédé reproductible, fiable et sensible.

Le procédé conforme à l'invention consiste essentiellement :

(a) à réaliser un conditionnement préalable de deshydratation de l'échantillon étudié de façon à réduire son taux d'hydratation pondéral à une valeur inférieure à 20 %,

(b) à disposer ledit échantillon entre les armatures d'un condensateur,

(c) à chauffer l'échantillon à une température dite de polarisation, comprise entre 30° C et une valeur inférieure à la température de dénaturation des protéines concernées,

(d) à soumettre ledit échantillon à un champ électrique statique compris entre $10^3$ V/m et $2.10^6$ V/m pendant une durée adaptée à l'établissement d'une polarisation électrique dudit échantillon,

(e) à refroidir l'échantillon jusqu'à une température inférieure à 10° C,

(f) à interrompre le champ électrique,

(g) à réchauffer l'échantillon à une vitesse d'échauffement inférieure à 40°/min jusqu'à une température supérieure à la température de dénaturation des protéines concernées,

(h) au cours dudit échauffement, à enregistrer en fonction de la température le courant de dépolarisation s'établissant entre les armatures du condensateur,

(i) dans une phase préalable d'étalonnage, à identifier et positionner le pic irréversible du courant de dépolarisation obtenu dans les mêmes conditions pour des protéines non modifiées d'un tissu normal du type concerné,

(j) et à comparer la variation du courant de dépolarisation de l'échantillon étudié à la variation dudit courant obtenue pour des protéines normales, et à détecter un éventuel abaissement d'au moins 10° C de la température du pic irréversible, caractéristique d'une altération des protéines de l'échantillon sous l'effet d'une absorption antérieure de drogue.

Le procédé de l'invention fait donc appel à la technique connue en soi des courants thermostimulés (CTS). On a pu constater que, dans les conditions de mise en oeuvre ci-dessus définies, un tissu musculaire soumis à une injection préalable de drogue avait un comportement différent quant à la position et à l'amplitude du pic de dépolarisation irréversible qui apparaît sur la courbe de dépolarisation. Les expérimentations ont montré que la température de ce pic était abaissée d'environ 40° et son amplitude divisée par un facteur de l'ordre de 4 à 10 dans le cas d'un tissu hormoné par rapport à un tissu normal de même nature. Dans ces conditions, il est ainsi facile de détecter l'injection antérieure de drogue chez l'animal et notamment de différencier une viande hormonée d'une viande saine.

Il est à noter que, dans un article rédigé par certains des inventeurs ("Innov. Techn. Biol. Med. ; Vol. 4 n° 4, 1985, p. 382-392, A. LAMURE et al., Etude du vieillissement du collagène par spectroscopie diélectrique très basse fréquence"), il est fait appel à la technique précitée des Courants Thermo-Stimulés pour étudier l'évolution des mouvements moléculaires réversibles dans un collagène sous l'effet du vieillissement ; dans ces travaux, cette technique était mise en oeuvre dans des conditions suffisamment douces pour éviter toute modification irréversible du collagène afin d'obtenir un résultat significatif sur les phénomènes dynamiques réversibles étudiés. Des études de ces phénomènes de vieillissement avaient d'ailleurs été effectuées antérieurement par des techniques biochimiques et avaient donné des résultats parfaitement significatifs, les travaux sus-évoqués par CTS n'ayant fait que confirmer ces résultats. Ces travaux ne fournissant aucun enseignement particulier pour résoudre le problème visé par l'invention de caractérisation de tissus soumis ou non à des injections préalables de drogue. En effet, il s'agit de phénomènes très différents (liaisons chimiques dans le cas de vieillissement, liaisons physiques dans le cas d'une altération hormonale) et de conditions de mise en oeuvre différentes (mettant en jeu des mécanismes réversibles) ; de plus, le fait que les techniques biochimiques traditionnelles ne donnent aucun résultat dans le problème visé par l'invention laisse prévoir que la technique CTS ne permettra de déceler les différences, puisque les travaux précités paraissaient montrer que les performances des deux types de techniques étaient équivalentes.

Les constatations des inventeurs sur le décalage en amplitude et en température du pic irréversible sont inédites et inattendues, d'autant que ce décalage est important, et rigoureusement reproductible.

Selon un mode de mise en oeuvre préféré, la phase préalable d'étalonnage (i) consiste à conditionner par deshydratation un échantillon témoin de tissu normal du type concerné, à le soumettre au protocole de manipulation (b) -(h) en vue d'enregistrer la variation du courant de dépolarisation de cet échantillon témoin, puis à renouveler ce protocole de manipulation (b) - (h) sur ce même échantillon témoin en vue d'obtenir un nouvel enregistrement de la variation du courant de dépolarisation, à comparer les deux enregistrements afin de déterminer le pic de courant présent sur le premier enregistrement et ayant une amplitude significativement plus faible sur le second, et à repérer la position en température de ce pic qui constitue le pic irréversible.

Cette mise en oeuvre permet de parfaitement identifier le pic irréversible afin de l'utiliser dans la caractérisation de l'échantillon étudié. Cet étalonnage peut être effectué, une fois pour toutes, pour chaque type de tissu ou viande à caractériser, en particulier dans les abattoirs où le procédé est appelé à être mis en oeuvre de façon systématique sur des types de viande prédéterminés (veaux d'âge donné, boeufs, porcs,...). Bien entendu, le procédé peut également être mis en oeuvre ponctuellement en laboratoire, avec un étalonnage au coup par coup.

Dans cette phase préalable d'étalonnage (i), l'on peut enregistrer sur un support (papier, support magnétique...) la courbe de variation du courant de dépolarisation obtenue à l'issue du premier protocole de manipulation, avec un repérage sur ladite courbe, du pic irréversible et de la température de ce pic. La comparaison (j) pour détecter un éventuel abaissement de la température du pic irréversible peut alors être effectuée par superposition de la courbe d'étalonnage sus-évoquée et de la courbe obtenue pour l'échantillon étudié. Cette superposition peut être faite visuellement, mais également par le calcul grâce à un programme approprié.

Par ailleurs, de préférence, le conditionnement préalable (a) de l'échantillon étudié ou de l'échantillon témoin consiste en une lyophilisation portant le taux d'hydratation dudit échantillon à une valeur de l'ordre de 10 %. Un tel conditionnement évite que le phénomène de décalage du pic irréversible soit partiellement ou totalement masqué par une relaxation provenant d'une quantité d'eau trop élevée dans le tissu.

Pour les mêmes raisons, l'échantillon étudié ou l'échantillon témoin sont de préférence placés dans une atmosphère inerte isolée, présentant initialement un taux d'hydratation prédéterminé, en particulier compris entre 3 et 10 %.

De plus, les conditions de mise en oeuvre suivantes paraissent fournir les meilleurs résultats et seront avantageusement choisies en pratique :
- température de polarisation comprise entre 40° C et 70° C,
- durée de polarisation à cette température comprise entre 10 minutes et 0,5 minute (en fonction inverse de la température de polarisation choisie),
- champ électrique ajusté à une valeur comprise entre $10^5$ et $10^6$ V/m,
- refroidissement de l'échantillon jusqu'à une température d'au moins -50° C à vitesse de refroidissement élevée, notamment par trempe au moyen d'azote liquide,
- réchauffement de l'échantillon à une vitesse d'échauffement de l'ordre de 7°/min. jusqu'à une température de l'ordre de 90° C à 100° C.

La description qui suit fournit un exemple de mise en oeuvre du procédé conforme à l'invention au moyen d'un dispositif connu en soi qui est schématisé sur les dessins ; sur ces dessins :
- la figure 1 est une coupe schématique axiale du dispositif,
- la figure 2 est un diagramme d'enregistrement des spectres de dépolarisation obtenus dans l'exemple de mise en oeuvre, dans le cas d'un échantillon témoin normal (courbes A et A') et d'un échantillon hormoné (courbe B).

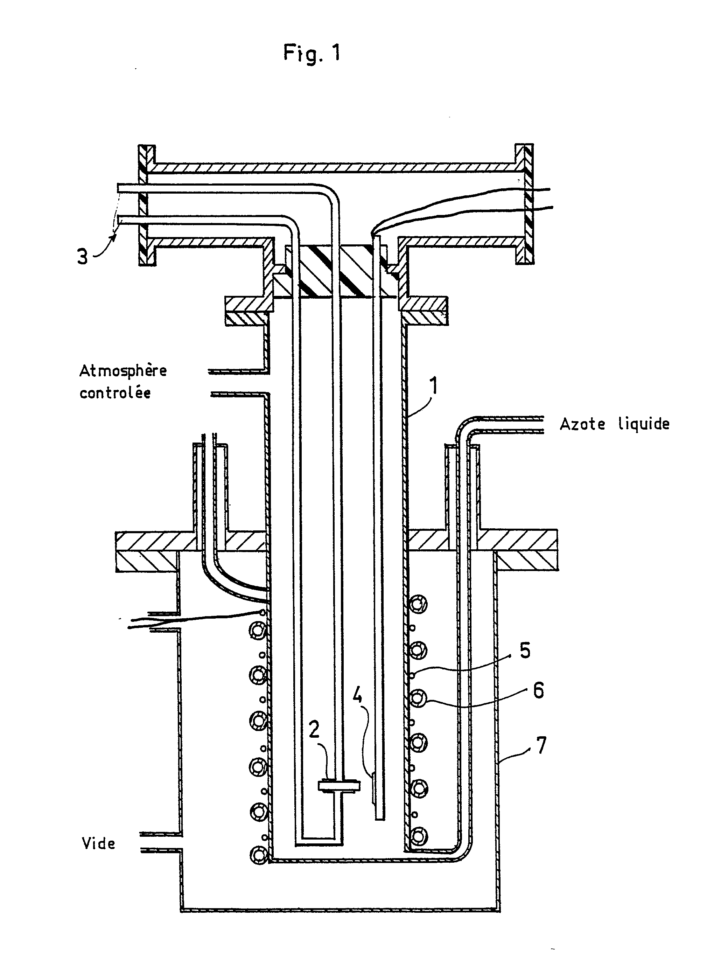

Le dispositif représenté à la figure 1 est une cellule de mesure de courants thermostimulés de type classique. Elle comprend essentiellement une enceinte étanche 1 soumise à une atmosphère contrôlée, en l'exemple air à 8 % d'humidité en poids. Dans cette enceinte est disposé un condensateur 2 dont les armatures permettent d'appliquer un champ électrique statique dans un échantillon intercalé entre celles-ci.

Les échantillons étudiés dans l'exemple présentent une masse supérieure à 10 mg (en l'exemple 40 mg), cette masse étant compactée entre les deux faces planes parallèles que forment les armatures du condensateur 2.

Ces armatures sont reliées à des conducteurs 3 pour leur mise sous tension ou pour la mesure des courants de dépolarisation. A cet effet, les conducteurs 3 sont connectés soit à une source de tension, soit au préamplificateur d'un électromètre dont la sensibilité est ajustée pour permettre de mesurer au moins $10^{-13}$A. Cet électromètre est en l'exemple du type "Keithley G42" avec sa sortie branchée sur l'entrée d'un micro-ordinateur recevant le signal issu de la sonde 4 en vue de l'enregistrement des courbes de courant en fonction de la température.

La température à proximité du condensateur 2 est mesurée au moyen d'une sonde platine 4.

Une résistance 5 permet d'assurer le chauffage, cependant qu'une circulation d'azote liquide 6 permet de réaliser un refroidissement brusque de l'enceinte. Une enceinte externe 7 isole l'ensemble de l'extérieur.

L'exemple décrit vise la caractérisation d'une viande de veau de 120 jours. P:our procéder à la phase préalable d'étalonnage, un veau témoin élevé sans hormone a permis de prélever un échantillon de tissu (en l'exemple du jarret : les essais ont montré que la caractérisation n'était pas influencée par la localisation du tissu prélevé). Cette viande est lyophilisée de façon traditionnelle ; à l'issue de cette opération, son taux d'hydratation est ramené à 10 % (en poids) et l'on en prélève 40 mg pour constituer l'échantillon témoin. Celui-ci est compacté au moyen d'une petite presse manuelle de façon à former un disque d'épaisseur de l'ordre de 500 microns et de diamètre de l'ordre de 0,8 cm.

Cet échantillon témoin est placé entre les armatures du condensateur 2, l'atmosphère de l'enceinte 1 étant de l'hélium.

Au moyen des résistances 5, l'échantillon est chauffé à une température de polarisation de 50° C. L'on applique alors au moyen des conducteurs 3 une différence de potentiel de 200 volts entre les armatures, ce qui correspond à un champ électrique d'environ $8.10^5$ V/m. Ce champ est maintenu pendant 2 minutes.

L'on arrête le chauffage et l'on fait circuler de l'azote liquide dans les conduits 6, ce qui provoque une trempe de l'échantillon et le porte à une température de l'ordre de -50° C, température à laquelle on arrête le refroidissement (à noter que la résistance 5 est utilisée pour stabiliser la température de trempe).

On interrompt alors le champ électrique et on branche le condensateur 2 à l'entrée de l'électromètre.

La résistance 5 est mise en oeuvre pour réchauffer l'échantillon de façon sensiblement linéaire avec une vitesse de 7°/min. jusqu'à une température de 100° C, supérieure à la température de dénaturation des protéines (qui est de l'ordre de 80° C pour la viande concernée).

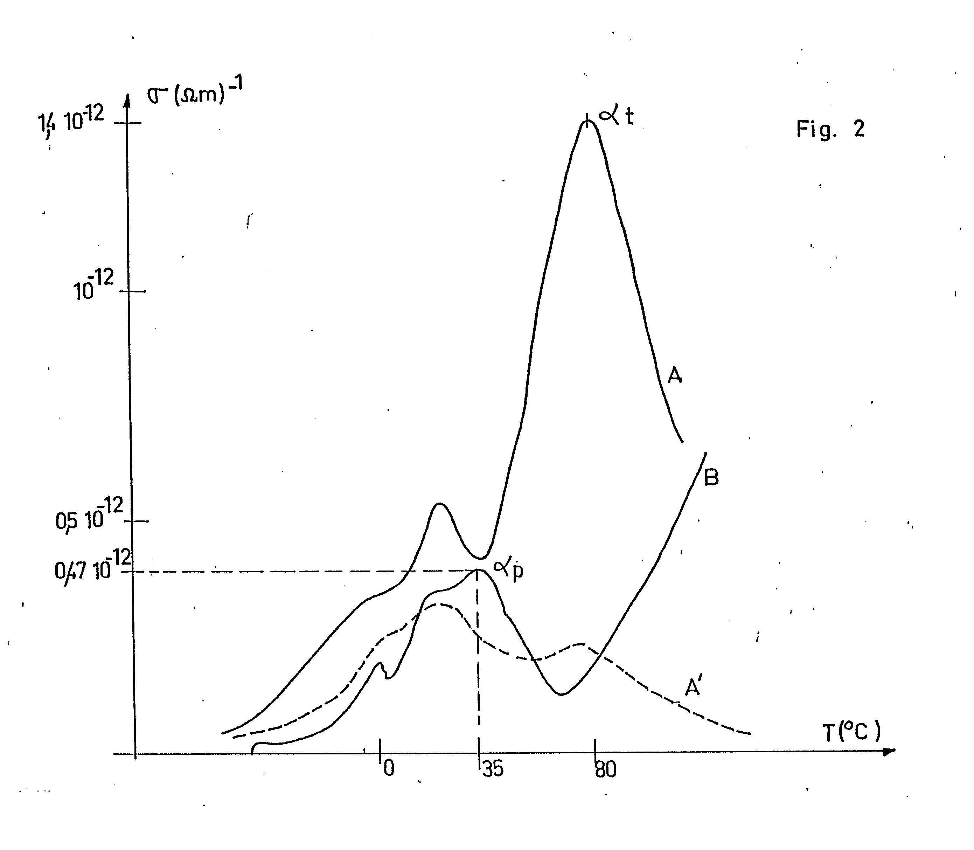

La courbe de courant est enregistrée et a été reportée sur le diagramme de la figure 2 (courbe A) en portant en coordonnée la conductivité électrique dynamique $\sigma = I. \left( \dfrac{e}{SV} \right)$,

S étant la surface de l'échantillon,
e son épaisseur,
et V la différence de potentiel entre les armatures.

(La prise en compte de $\sigma$ au lieu du courant I permet de s'affranchir de la géométrie de l'échantillon ; il est bien entendu que par enregistrement du courant de dépolarisation, on entend l'enregistrement de toute grandeur qui en est directement fonction).

On observe sur la courbe A un pic $\alpha_t$ très majoritaire vers 80° C ($\mp$ 5° C) pour une valeur de $\sigma$ de l'ordre de $1,4.10^{-12}$ $(\Omega. m)^{-1}$.

Afin de vérifier le caractère irréversible de ce pic, l'on effectue sur le même échantillon une deuxième série de manipulation immédiatement après la première, après avoir ramené l'échantillon à la température de polarisation de 50° C (durée : 2 minutes, champ électrique, refroidissement à -50° C, interruption du champ, réchauffement à 100° C avec enregistrement). Les conditions de manipulation sont les mêmes que précédemment.

L'on obtient alors la courbe A' dessinée en traits discontinus à la figure 2. L'on constate que le pic $\alpha_t$ a disparu (alors que les autres pics à température plus basse demeurent) : le pic $\alpha_t$ est le pic irréversible à prendre en compte pour la caractérisation (pic associé au mécanisme de dénaturation des protéines qui se produit vers 80° C pour une viande normale).

Ensuite a lieu la phase de caractérisation proprement dite qui est effectuée par des séries d'expérimentation portant en premier lieu sur des viandes de veau de 120 jours non hormonées : l'on constate que la courbe obtenue est indépendante de l'animal et coïncide à $\mp$ 5° en température avec la courbe A (par contre, les valeurs de $\sigma$ peuvent varier davantage d'un essai à l'autre).

Le même protocole est ensuite appliqué à une viande d'un veau (120 jours) aui a été traité au moyen d'anabolisants. (zéralone et trembolone) aux doses habituelles de traitement pratiqués en FRANCE avant 1986.

Les conditions de préparation de l'échantillon et de caractérisation sont analogues aux précédentes (compactage, chauffage à 50° C, polarisation à $8.10^5$ V/m, durée de polarisation de 2 minutes, trempe jusqu'à -50° C, interruption du champ, réchauffement à 7°/minute jusqu'à 100° C et enregistrement).

La courbe obtenue a été reportée en B à la figure 2. Le pic $\alpha_t$ n'est plus observé à 80° C et est remplacé par un pic $\alpha_h$ à 35° C avec une conductivité $\sigma$ de l'ordre de 0,4 $10^{-12}$. Des séries d'expériences avec des viandes hormonées (plus de 20 échantillons) redonnent les mêmes résultats à $\frac{}{+}$ 5° C en température (la conductivité du pic

$\alpha_h$ est toujours très inférieure à celle du pic $\alpha_t$ de la courbe témoin A et présente une dispersion plus grande).

Ainsi l'abaissement du pic $\alpha_t$ vers $\alpha_h$ permet de conclure au caractère hormoné de la viande. Le phénomène utilisé dans cette caractérisation consiste donc en une modification des conditions d'apparition du mécanisme de dénaturation des protéines constitutives du tissu musculaire, sous l'effet thermique. En pratique, l'on a évalué à environ 10° C l'abaissement nécessaire pour pouvoir conclure de façon fiable à une injection préalable de drogue.

**Revendications**

1/ - Procédé de caractérisation des protéines d'un échantillon de tissu musculaire en vue de détecter une éventuelle absorption antérieure de drogue ayant entraîné une altération des protéines dudit tissu, caractérisé en ce qu'il consiste :

(a) à réaliser un conditionnement préalable de deshydratation de l'échantillon étudié de façon à réduire son taux d'hydratation pondéral à une valeur inférieure à 20 %,

(b) à disposer ledit échantillon entre les armatures d'un condensateur,

(c) à chauffer l'échantillon à une température dite de polarisation, comprise entre 30° C et une valeur inférieure à la température de dénaturation des protéines concernées,

(d) à soumettre ledit échantillon à un champ électrique statique compris entre $10^3$ V/m et $2.10^6$ V/m pendant une durée adaptée à l'établissement d'une polarisation électrique dudit échantillon,

(e) à refroidir l'échantillon jusqu'à une température inférieure à 10° C,

(f) à interrompre le champ électrique,

(g) à réchauffer l'échantillon à une vitesse d'échauffement inférieure à 40°/min jusqu'à une température supérieure à la température de dénaturation des protéines concernées,

(h) au cours dudit échauffement, à enregistrer en fonction de la température le courant de dépolarisation s'établissant entre les armatures du condensateur,

(i) dans une phase préalable d'étalonnage, à identifier et positionner le pic irréversible du courant de dépolarisation obtenu dans les mêmes conditions pour des protéines non modifiées d'un tissu normal du type concerné,

(j) et à comparer la variation du courant de dépolarisation de l'échantillon étudié à la variation dudit courant obtenue pour des protéines normales, et à détecter un éventuel abaissement d'au moins 10° C de la température du pic irréversible, caractéristique d'une altération des protéines de l'échantillon sous l'effet d'une absorption antérieure de drogue.

2/ - Procédé selon la revendication 1, caractérisé en ce que la phase préalable d'étalonnage (i) consiste à conditionner par deshydratation un échantillon témoin de tissu normal du type concerné, à le soumettre au protocole de manipulation (b) - (h) en vue d'enregistrer la variation du courant de dépolarisation de cet échantillon témoin, puis à renouveler immédiatement ce protocole de manipulation (c) -(h) sur ce même échantillon témoin en vue d'obtenir un nouvel enregistrement de la variation du courant de dépolarisation, à comparer les deux enregistrements afin de déterminer le pic de courant présent sur le premier enregistrement et ayant une amplitude significativement plus faible sur le second, et à repérer la position en température de ce pic qui constitue le pic irréversible.

3/ - Procédé selon la revendication 2, caractérisé en ce que :
- dans la phase préalable d'étalonnage (i), l'on enregistre sur un support la courbe de variation du courant de dépolarisation obtenue à l'issue du premier protocole de manipulation, avec un repérage sur ladite courbe, du pic irréversible et de la température de ce pic,
- la comparaison (j) pour détecter un éventuel abaissement de la température du pic irréversible est effectuée par superposition de la courbe d'étalonnage sus-évoquée et de la courbe obtenue pour l'échantillon étudié.

4/ - Procédé selon l'une des revendications 1, 2 ou 3, dans lequel chaque enregistrement de courant de dépolarisation est effectué en branchant les armatures du condensateur à l'entrée d'un électromètre de sensibilité adaptée pour permettre de mesurer au moins $10^{-13}$A.

5/ - Procédé selon l'une des revendications 1, 2, 3 ou 4, dans lequel l'échantillon étudié ou l'échantillon témoin est préparé pour présenter une masse au moins égale à 10 mg et est compactée entre deux faces planes parallèles.

6/ - Procédé selon l'une des revendications 1, 2, 3, 4 ou 5, dans lequel le conditionnement préalable (a) de l'échantillon étudié ou de l'échantillon témoin consiste en une lyophilisation portant le taux d'hydratation dudit échantillon à une valeur de l'ordre de 10 %.

7/ - Procédé selon l'une des revendications 1, 2, 3, 4, 5 ou 6, dans lequel (b) l'on dispose l'échantillon étudié ou l'échantillon témoin dans un condensateur placé dans une atmosphère inerte isolée, présentant initialement un taux d'hydratation prédéterminé.

8/ - Procédé selon l'une des revendications précédentes, caractérisé en ce que (c) l'on chauffe l'échantillon étudié ou l'échantillon témoin à une température de polarisation comprise entre 40° C et 70° C et (d) l'on soumet ledit échantillon chauffé à ladite température au champ électrique pendant une durée comprise entre 10 minutes et 0,5 minute, en fonction inverse de la température de polarisation choisie.

9/ - Procédé selon la revendication 8, dans lequel (d) le champ électrique est ajusté à une valeur comprise entre $10^5$ et $10^6$ V/m.

10/ - Procédé selon l'une des revendications précédentes, dans lequel (e) l'échantillon est refroidi jusqu'à une température d'au moins - 50° C à vitesse de refroidissement élevée.

11/ - Procédé selon la revendication 10, dans lequel (e) l'échantillon est refroidi par une trempe au moyen d'azote liquide.

12/ - Procédé selon l'une des revendications précédentes, dans lequel (g) l'on réchauffe l'échantillon étudié ou l'échantillon témoin à une vitesse d'échauffement de l'ordre de 7°/min jusqu'à une température de l'ordre de 90° C à 100° C.

# Fig. 1

Atmosphère
controlée

Azote liquide

Vide

3

1

2

4

5

6

7

Fig. 2

EP 0 346 968 A2